# EUROPEAN PATENT APPLICATION

(11) **EP 4 553 850 A1**
(43) Date of publication of application: **14.05.2025**
(21) Application number: 23897195.6
(22) Date of filing: 11.09.2023
(51) Int. Cl.: G16H 50/30

(54) **HEALTH MANAGEMENT ASSISTANCE SYSTEM, HEALTH MANAGEMENT ASSISTANCE METHOD, AND PROGRAM**

(30) Priority: 30.11.2022 JP 2022192066
(71) Applicant: Foundation of Learning Health Society Institute, Nagoya-shi, Aichi 450-0003 (JP)
(72) Inventor: FUKUSHIMA, Masanori, Nagoya-shi, Aichi 458-0045 (JP); TANZAWA, Kazumasa, Tokyo 154-0021 (JP); KIKUCHI, Takayuki, Itinomiya, Aichi 491-0039 (JP)
(74) Representative: KATZAROV S.A.
(86) International application number: PCT/JP2023/032974
(87) International publication number: WO 2024/116536

(57) **Abstract**

A health management support system includes: an obtainer (11) that obtains, from each terminal device of users, personal data including physical information, social information, and mental information on the user and terminal usage information indicating a usage status of the terminal device of the user, at predetermined time intervals; a storage device (12) that accumulates population data including the personal data on each of the users; a prediction model unit (13) that predicts, from time-series personal data accumulated in the storage device (12) for a predetermined time period, a future risk of one user among the users, using a prediction model (13a) for predicting the future risk by applying the population data to the personal data that includes temporal changes; and a provider (14) that outputs, to the terminal device of the one user, provision information generated based on the future risk predicted and indicating health management support.

## Description

### [Technical Field]

The present disclosure relates to a health management support system, a health management support method, and a program.

### [Background Art]

The percentage of the elderly aged 65 and over in Japan was about 30% in 2019, but is estimated to reach about 40% by 2060. With this increase, the number of persons who will become in need of support or nursing care is estimated to rise, posing concerns about an increase in the social burden of caring the elderly.

Under such circumstances, there is a need to inhibit the number of elderly persons who will become in need of support or nursing care.

To avoid becoming in need of nursing care in the future, for example, an evaluation of the risk of locomotive syndrome is considered important together with an early detection of locomotive syndrome.

To cope with this, a system is proposed that evaluates the risk of locomotive syndrome, using a plurality of wearable sensors (see, for example, Patent Literature (PTL) 1).

According to the system disclosed in PTL 1, it is possible to evaluate the risk of locomotive syndrome on the basis of variations in measurement data on walking motions that has been obtained using the plurality of wearable sensors, and provide feedback to the test subject.

### [Citation List]

### [Patent Literature]

[PTL 1] Japanese Unexamined Patent Application Publication No. 2018-114021

### [Summary of Invention]

### [Technical Problem]

However, the use of the system disclosed in PTL 1 imposes constrains such as that the measurement devices need to be attached on a plurality of specified portions on the body and need to be implemented in the form of an application in a mobile terminal such as a smartphone. For this reason, the number of users of such system is likely to be limited, and so is the number of persons in the stage before becoming in need of support or nursing care. As a result, it is highly likely that the number of elderly persons who will become in need of support or nursing care in the future fails to decrease. Also, to avoid becoming in need of nursing care in the future, not only an early detection of locomotive syndrome, but also an early detection of frailty and cognitive decline is important.

The present disclosure has been conceived in view of the above circumstances, and it is an aim of the present disclosure to provide a health management support system and so forth capable of providing health management support that enables each of a plurality of users to avoid becoming in need of support or nursing care in the future.

### [Solution to Problem]

To solve the above problem, a health management support system according to an aspect of the present disclosure includes: an obtainer that obtains personal data from each terminal device of a plurality of users at predetermined time intervals, the personal data including gait information on the user and terminal usage information indicating a usage status of the terminal device of the user; a storage device that accumulates population data including the personal data on each of the plurality of users obtained by the obtainer; a prediction model unit that predicts a future risk of one user becoming in need of nursing care or support, using a prediction model, from time-series personal data that is the personal data on the one user accumulated in the storage device for a predetermined time period, the one user being included in the plurality of users, the prediction model being a model for predicting the future risk by applying the population data to the personal data that includes temporal changes; and a provider that outputs, to the terminal device of the one user, provision information that is generated based on the future risk predicted and indicates health management support for the one user.

Note that these general and specific aspects may be implemented using a system, a method, an integrated circuit, a computer program, or a computer-readable recording medium such as a CD-ROM, or any combination of systems, methods, integrated circuits, computer programs, or recording media.

### [Advantageous Effects of Invention]

According to the health management support system and so forth of the present disclosure, it is possible to provide health management support that enables each of a plurality of users to avoid becoming in need of support or nursing care in the future simply through a daily use of a mobile terminal of such user, without having to install any special software in the mobile terminal.

### [Brief Description of Drawings]

[FIG. 1]

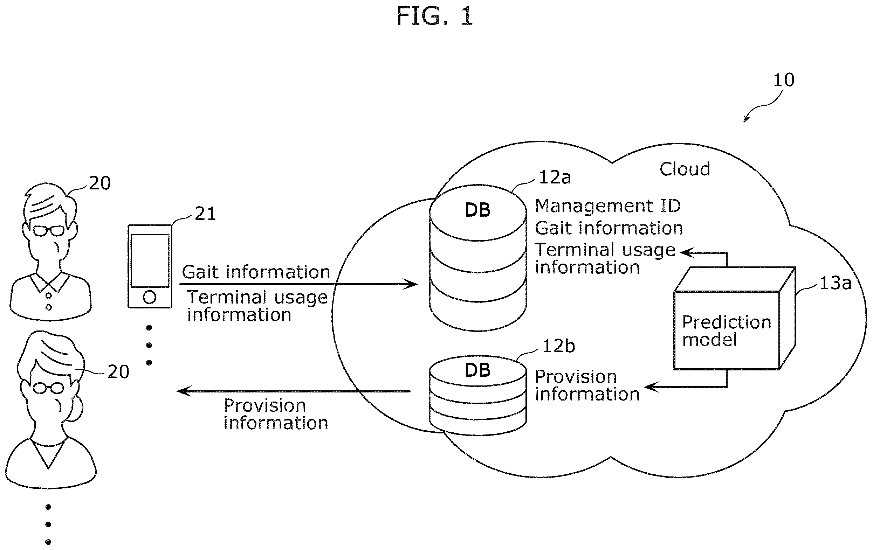

   FIG. 1 is a diagram conceptually showing a health management support method according to Embodiment 1.
[FIG. 2]

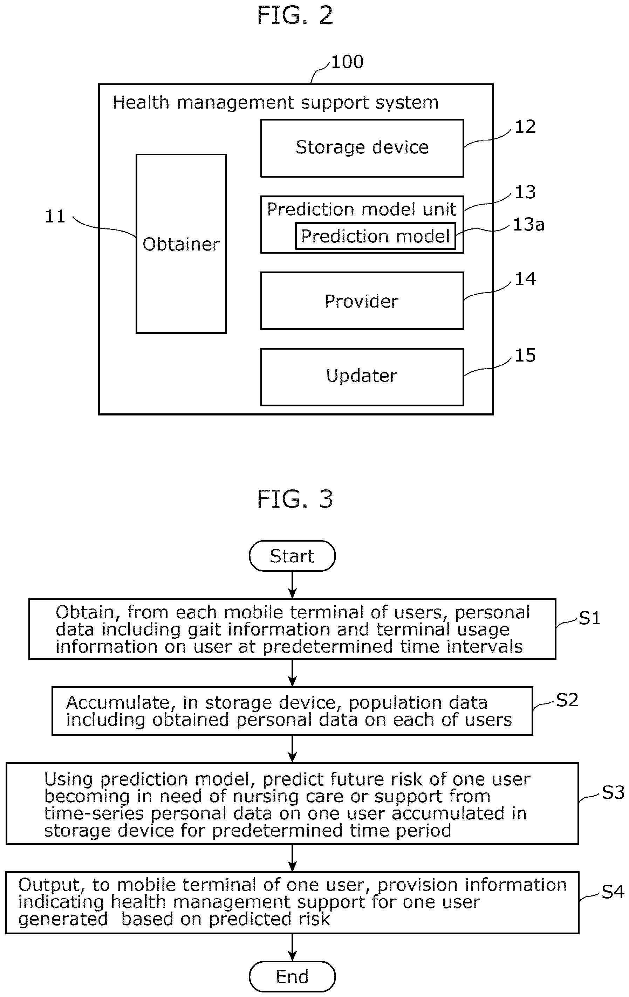

   FIG. 2 is a block diagram showing an example of the configuration of a health management support system according to Embodiment 1.
[FIG. 3]
   FIG. 3 is a flowchart showing an example of the processing of the health management support method performed by the health management support system according to Embodiment 1.
[FIG. 4]

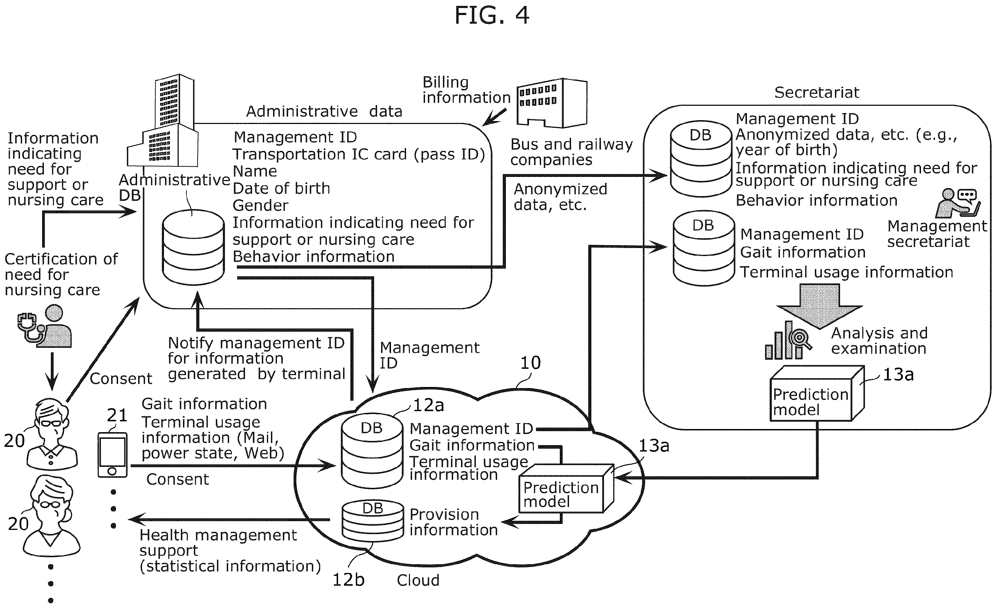

   FIG. 4 is a diagram conceptually showing a health management support method according to a working example of Embodiment 1.
[FIG. 5]

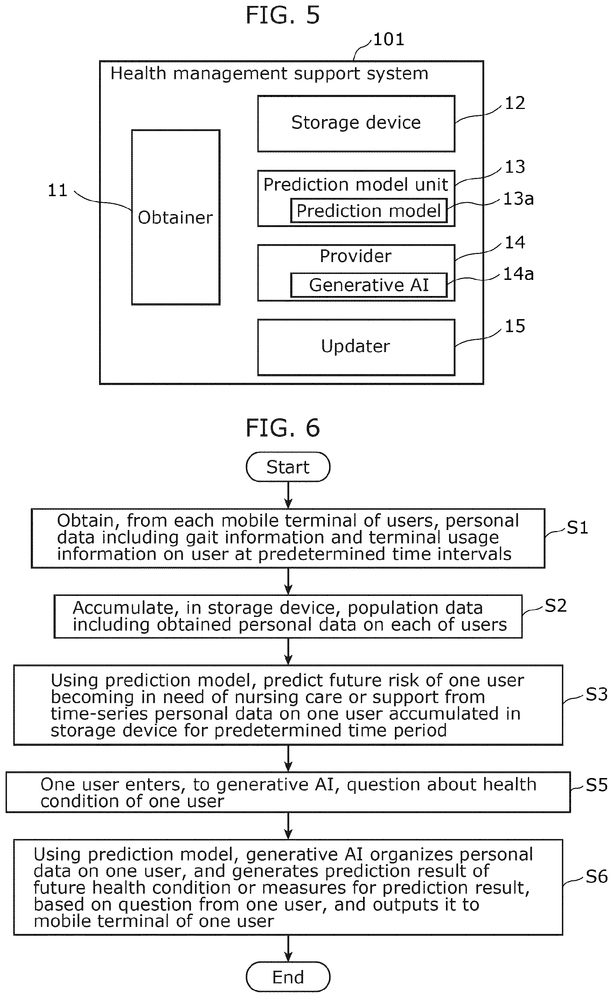

   FIG. 5 is a block diagram showing an example of the configuration of a health management support system according to Embodiment 2.
[FIG. 6]
   FIG. 6 is a flowchart showing an example of the processing of the health management support method performed by the health management support system according to Embodiment 2.
[FIG. 7]

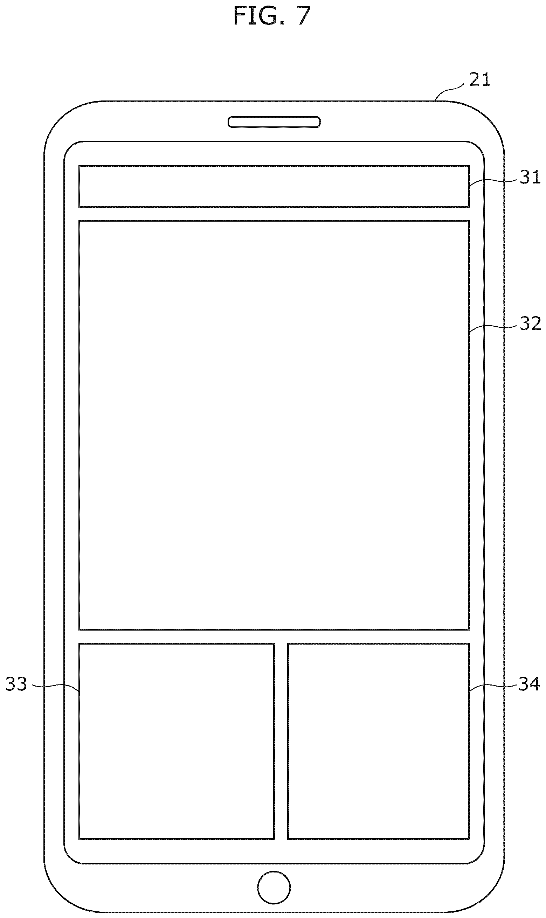

   FIG. 7 is a diagram for describing input/output regions on a mobile terminal.
[FIG. 8A]

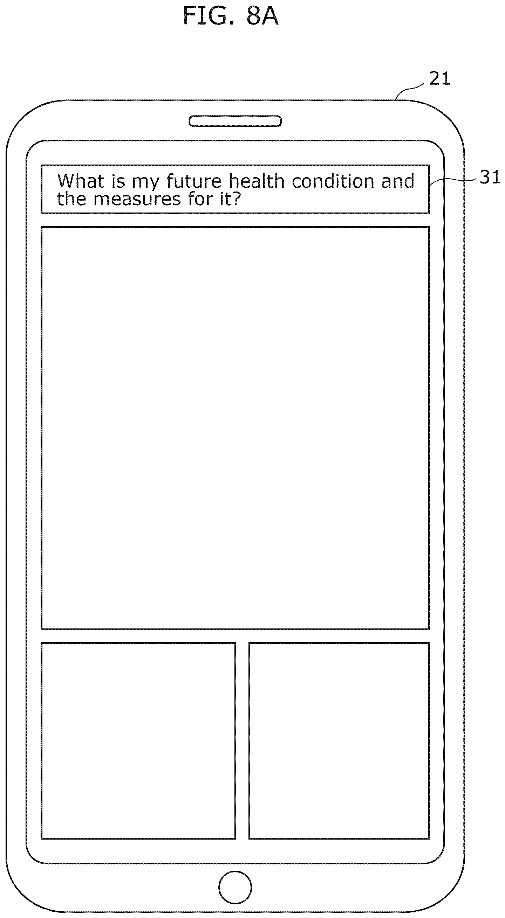

   FIG. 8A is a diagram for describing the input/output regions on the mobile terminal when one user enters a question to the mobile terminal.
[FIG. 8B]

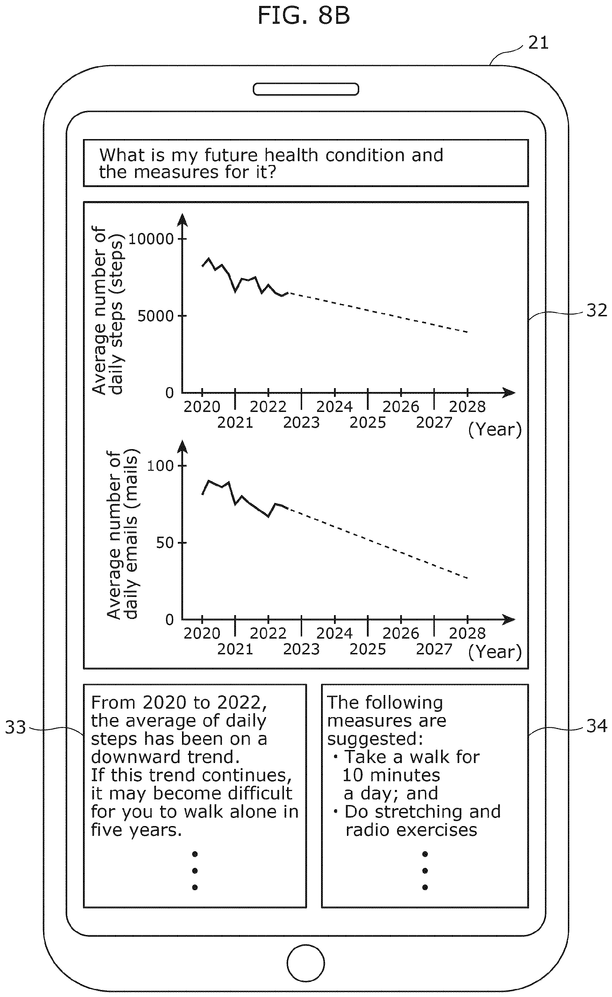

   FIG. 8B is a diagram for describing the input/output regions on the mobile terminal when a generative AI responds to the question from the one user.

### [Description of Embodiments]

Each of the exemplary embodiments described below shows a specific example of the present disclosure. The numerical values, shapes, elements, steps, the processing order of the steps, etc. shown in the following exemplary embodiments are mere examples, and therefore do not limit the scope of the present disclosure. Therefore, among the elements in the following exemplary embodiments, those not recited in any one of the independent claims are described as optional elements. Also, the details of each of all the embodiments can be combined with each other.

### [Embodiment 1]

The following describes a health management support method and a health management support system according to Embodiment 1 with reference to the drawings.

### [1 Conceptual Diagram of Health Management Support Method]

FIG. 1 is a diagram conceptually showing the health management support method according to Embodiment 1.

As shown in FIG. 1, in the health management support method according to Embodiment 1, data obtained by each of a plurality of users 20 through a daily use of mobile terminal 21 is obtained from mobile terminal 21 of such user 20 and accumulated in, for example, cloud 10 (DB 12a) on a daily basis. The data accumulated in cloud 10 (DB12a) on a daily basis is, for example, population data, which is a collection of items of personal data. The personal data is, for example, data such as gait information on each of the plurality of users 20 and terminal usage information indicating the usage status of the mobile terminal of each of the plurality of users 20.

Here, an example of each of the plurality of users 20 is, but not limited to, an elderly person. Each of the plurality of users 20 may thus be any person at a certain age and over. Mobile terminal 21 is, for example, a wearable device such as a smartphone and a smartwatch that can be carried around by user 20 and communicate with cloud 10. Note that mobile terminal 21 is one example of the terminal device. The terminal device may also be a stationary computer. More specifically, a wearable device may store the personal data, which is data such as the gait information on each of the plurality of users 20 and the terminal usage information indicating the usage status of the mobile terminal of each of the plurality of users 20, and transmit the personal data to the stationary computer.

In recent years, many people have been using mobile terminals such as smartphones. In mobile terminals such as smartphones, it is easy to use email software, social networking service (SNS)-enabled software, Web-enabled software, healthcare applications, etc. Under such circumstances, many people daily use email software and healthcare applications on smartphones, and personal data that can be said to be daily lifestyle data on an individual obtained through such daily use of email software and a healthcare application is stored on the smartphone.

In Embodiment 1, personal data obtained by each of the plurality of 20 users, or the foregoing many people, through a daily use of existing software such as email software and a healthcare application using his/her own mobile terminal 21, is stored in cloud 10 (DB 12a) on a daily basis. Various kinds of data can be conceived as the personal data obtained through a daily use of the healthcare application, but it suffices if data showing the relation of at least two of the following items of data (gait information) is available: a daily walking time; a daily number of steps; a walking speed; a stride length; the number of stairs climbed; a walking distance; and others. Also, the personal data obtained through a daily use of existing software such as email software may be, for example, data including at least one of the following items of data: the frequency with which user 20 exchanges emails using mobile terminal 21; the length of sentences included in emails; the number of persons with whom user 20 exchanges emails; and the usage rate of various software and applications in mobile terminal 21. Note that a link between the data obtained through a daily use of existing software such as email software and the software and applications in use may be any information related to interactions with another person performed by user 20 using mobile terminal 21, and the foregoing description is just one example. Also, the personal data is described above as being accumulated in cloud 10 (DB 12a) on a daily basis, but the present embodiment is not limited to this; the personal data may be accumulated at predetermined time intervals, such as every other day, so long as it is possible to check a short-term and long-term change amount and the speed of change.

In the health management support method according to Embodiment 1, the future risk of becoming in need of nursing care or support is predicted, using prediction model 13a, from the personal data accumulated in cloud 10 on a daily basis, and provision information indicating, for example, health management support is generated, on the basis of the predicted risk. Prediction model 13a enables the prediction of the foregoing risk by applying the population data to the personal data that includes temporal changes. The provision information generated is stored in cloud 10 (DB 12b) and transmitted to mobile terminal 21 of the corresponding user 20.

Here, the states before becoming in need of nursing care or support include the state of frailty, the state of locomotive syndrome, and the state of cognitive decline. The state of frailty is a state in which resistance has been weakened and physical strength has been reduced with aging. The state of locomotive syndrome is a state in which the functions of locomotive organs such as joints have declined, making it difficult to move around. The state of cognitive decline is a state in which the abilities relating to cognitive functions, such as comprehension, judgment, memory, and language comprehension, have declined. Stated differently, the states of frailty, locomotive syndrome, and cognitive decline can be said to be states that impair life functions, impair healthy life expectancy, and increase the risk of becoming in need of nursing care. It is well known that a preventive or early care for the states of frailty, locomotive syndrome, and cognitive decline is important because these states often occur and advance without a person being aware of it. At the same time, it is also known that appropriate measures taken against these states can reduce the risk of becoming in need of nursing care and return the state of the person to the original healthy state. It is also becoming clear what kind of measures should be taken for a person in the state of frailty, locomotive syndrome, and cognitive decline to slow the development of these states, or to return the state of such person to the original state.

In Embodiment 1, the future risk of becoming in need of nursing care or support, for example, is, but not limited to, information indicating that a person has a sign of getting locomotive syndrome, frailty, or dementia (risk factor information). Such risk may also be information indicating that the person is likely to fall into such states (predictive factor information). The foregoing risk may also be information indicating that the person is in the state of locomotive syndrome, frailty, or cognitive decline, and is likely to become in need of nursing care or support if the state continues to develop (prognostic factor information).

The health management support may also be, for example, information indicating measures to inhibit the development into at least one of frailty, locomotive syndrome, or dementia. The measures may include, but are not limited to, recommending to treat or manage an injury or a disease that can be the cause of the development into at least one of frailty, locomotive syndrome, or dementia, or to do exercise that inhibits the development into at least one of these. Such measures may further include recommending appropriate improvements in lifestyle, such as recommending appropriate diet and good sleep. Note that a considerable individual effort is required to prevent the development of frailty, locomotive syndrome, and dementia. At present, it is known that medication can inhibit the development of these but cannot stop them to develop. In addition, it is becoming clear that appropriate lifestyle improvements, such as doing appropriate exercise and having appropriate diet and good sleep, are essential to prevent the development of frailty, locomotive syndrome, and dementia.

As described above, the health management support method according to Embodiment 1 enables user 20 to obtain the provision information, such as health management support, simply through a daily use of mobile terminal 21, without having to install any special software in mobile terminal 21 of such user 20. With this, in response to the reception of the provision information, user 20 becomes aware of the need for changing his/her behavior to avoid the risk and can change his/her behavior. It is thus possible for user 20 to avoid becoming in need of support or nursing care in the future.

### [2 Configuration of Health Management Support System]

FIG. 2 is a block diagram showing an example of the configuration of health management support system 100 according to Embodiment 1. Health management support system 100 shown in FIG. 2 is realized, using, for example, a computer that includes a processor (microprocessor), a memory, a communication interface, etc. Such health management support system 100 includes obtainer 11, storage device 12, prediction model unit 13, provider 14, and updater 15. Health management support system 100 shown in FIG. 2 realizes, for example, cloud 10 shown in FIG. 1, using the elements thereof in part or in whole.

### [2.1 Obtainer 11]

Obtainer 11 is, for example, a communication interface and is capable of communicating with mobile terminal 21 of each of the plurality of users 20. Obtainer 11 obtains, from each mobile terminal 21 of the plurality of users 20, the personal data including the gait information on such user 20 and the terminal usage information indicating the usage status of mobile terminal 21 of such user 20, at predetermined time intervals.

As is described above, an example of each of the plurality of users 20 is, but not limited to, an elderly person, and thus each of the plurality of users 20 may be any person at a certain age and over. Mobile terminal 21 is, for example, a smartphone or a smartwatch, and any wearable device that can be carried around by user 20 and communicate with cloud 10 suffices.

In Embodiment 1, obtainer 11 obtains the gait information and the terminal usage information as the personal data, which can be said to be daily lifestyle data on an individual obtained by each of the plurality of users 20 through a daily use of existing software such as email software, healthcare application, etc., using mobile terminal 21, and accumulates the obtained personal data in storage device 12, together with a management ID. The management ID is, for example, an identifier to uniquely identify user 20. The details of the gait information and the terminal usage information are as described above, and thus will not be described here.

### [2.2 Storage Device 12]

Storage device 12 is realized, using, for example, a hard disk drive (HDD) or a solid state drive (SSD). Storage device 12 stores the population data that includes the personal data on each of the plurality of 20 users obtained by obtainer 11. Storage device 12 may be disposed, for example, in cloud 10 shown in FIG. 1, and may have a plurality of databases such as DB12a and DB12b. In this case, for example, that the gait information and the terminal usage information are preferably stored in DB 12a as the population data together with the management ID, and the provision information and others to be described later are preferably stored in DB 12b together with the management ID.

### [2.3 Prediction Model Unit 13]

Prediction model unit 13 includes, for example, a communication interface, a memory, and a processor (microprocessor). Prediction model unit 13 can realize the prediction function that uses prediction model 13a by means of the processor executing a predetermined program stored in the memory. Prediction model unit 13 may construct prediction model 13a and use such prediction model 13a, or may obtain constructed prediction model 13a and use such prediction model 13a. Using prediction model 13a, prediction model unit 13 predicts the future risk of one user 20, among the plurality of users 20, becoming in need of nursing care or support, from time-series personal data, which is the personal data on such one user 20 accumulated in storage device 12 for a predetermined time period.

For example, prediction model unit 13 may extract, as the personal data, physical information indicating a physical condition, social information indicating a social contact condition, and mental information indicating a mental condition from the gait information and the terminal usage information on the one user 20 accumulated in storage device 12 for the predetermined time period. Then, using prediction model 13a, prediction model unit 13 may predict the risk of the one user 20 from the physical information, the social information, and the mental information that have been extracted. For example, the physical information is the gait information on the one user 20. The gait information may include at least one of a daily walking time, a daily number of steps, or a walking speed. The social information is information on interactions with others performed by the one user 20, using mobile terminal 21. As described above, the information on the interactions may include, for example, at least one of the frequency with which the one user 20 exchanges emails using mobile terminal 21, the length of sentences included in the emails, or the number of persons with whom the one user 20 exchanges the emails. Also, the mental information may include at least one of sleep information or mindfulness information on the one user 20.

As described above, the future risk is, for example, information indicating that the one user 20 has a sign of getting locomotive syndrome, frailty, or dementia (risk factor information). The future risk may also be, for example, information indicating that the one user 20 is likely to get locomotive syndrome, frailty, or dementia (predictive factor information) if nothing is done for it. The risk may also be, for example, information indicating that the one user 20 is in the state of locomotive syndrome, frailty, or cognitive decline, and is likely to become in need of nursing care or support if the condition continues to develop (prognostic factor information).

Note that prediction model unit 13 may predict the risk of the one user 20 by checking, against prediction model 13a, the physical information, the social information, and the mental information that have been extracted, and examining the risk factor information, the predictive factor information, or the prognostic factor information. Using prediction model 13a, prediction model unit 13 may further generate statistical information on one user 20, among the plurality of users 20, from the time-series gait information and terminal usage information on such one user 20 accumulated in storage device 12 for the predetermined time period, and output the generated statistical information to provider 14. In the foregoing example, the statistical information is information representing, in a graphical form, how the daily lifestyle condition, the physical condition, the social activity condition, and the mental or psychological condition of user 20 change (especially decline) over time, but the present embodiment is not limited to this.

Here, prediction model 13a is a model that enables the prediction of the risk of an individual, by applying the population data to the personal data that includes temporal changes. Prediction model 13a is constructed (generated), for example, from the population data that includes the gait information and the terminal usage information on each of a predetermined number of users 20 accumulated in storage device 12 for a predetermined time period in the past, and diagnostic data in medical institutions, etc., on the basis of which the predetermined number of users 20 were certified as in need of nursing care or support. For example, prediction model 13a may be constructed from the physical information, the social information, and the mental information on the predetermined number of users extracted from the population data, and the diagnostic data.

Also, prediction model 13a may indicate variables that represent temporal changes in the physical information, the social information, and the mental information on the predetermined number of users and that serve as the risk factor information, the predictive factor information, or the prognostic factor information in relation to the diagnostic data, but the present embodiment is not limited to this. Prediction model 13a may be constructed through machine learning or may be constructed as a model obtained by formulating the population data obtained by statistical processing. Stated differently, prediction model 13a may be constructed in any manner, so long as it is possible with such constructed prediction model 13a to predict the future risk of user 20 becoming in need of nursing care or support from chronological data (time-series data) of a certain time period, including the gait information and the terminal usage information on such user 20. Note that, using prediction model 13a, for example, the population data that can be said to be big data obtained by accumulating the personal data on each of the plurality of users for a predetermined time period may be matched with the personal data of a certain time period that includes temporal changes. In this case, prediction model 13a enables the prediction of the future risk of a target individual becoming in need of nursing care or support from the diagnostic data of the personal data on others to be matched with the personal data included in the population data. Alternatively, prediction model 13a may be subjected to machine learning and the personal data of a certain time period is entered to such machine-learned prediction model 13a to predict the future risk of such target individual becoming in need of nursing care or support. The foregoing prediction methods are examples, and thus prediction methods using prediction model 13a are not limited to the cases described above.

As described above, many users 20 use mobile terminals 21 such as smartphones, and such many users 20 have data items stored in mobile terminals 21 that are obtained through a daily use of existing software, such as email software and healthcare applications, using mobile terminals 21. The inventors have conducted intensive studies to find that information on the daily lifestyle condition, the physical condition, the social activity condition, and the mental or psychological condition of each user 20 are stored in mobile terminals 21. The inventors have also found that it is possible to chronologically observe these items of information by accumulating such items of information, and to check how the foregoing conditions of each user 20 change (especially decline) over time by representing these items of information in a graphical form. The inventors have further found that it is possible to examine the information on the physical condition, the social activity condition, and the mental or psychological condition of each user 20 by, for example, checking the population data obtained by performing statistical processing on the accumulated items of information on the plurality of users 20 against the accumulated data on user 20 (personal data). The results of the studies show that the gait information and the terminal usage information, such as information on email exchanges, serve as important information in predicting the future risk of becoming in need of nursing care or support. For example, it has been found possible to examine (observe) the decline in the physical functions from temporal changes in the daily walking time, the number of steps, or the walking speed of user 20 who uses a healthcare application. It has been found also possible to examine the decline in social contact of user 20 by examining temporal changes in, for example, the frequency with which user 20 exchanges emails. For those who make Web searches, for example, it has been also found possible to examine the degree of interest in matters from the number of conducting searches, and to further know their interests from types of keywords used for the searches. As described above, it has been found that the examination of important information among the items of information accumulated enables the detection of a sign of physical frailty, mental frailty, and social frailty, as the future risk of becoming in need of nursing care or support.

From the above, the inventors have found that it is possible to construct a model (prediction model) that enables the prediction of the future risk of an individual becoming in need of nursing care or support by applying the population data to the personal data that includes temporal changes.

Note that with the implementation of the Basic Act on the Advancement of Public and Private Sector Data Utilization, it is becoming possible for many municipalities to use administrative data. Under such circumstances, when the administrative data managed by an administrative body is available in constructing prediction model 13a, prediction model 13a may be constructed using such administrative data.

Here, the administrative data is outcome data on individual persons, and includes, for example, data on the certification for need for support or nursing care, mortality data, and medical checkup data. The administrative data can further include information on the usage status of administrative services. The usage status of the administrative services is assumed to be, for example, the usage status of public transportation, which can be used as behavior information on a plurality of persons in daily life. The usage status of public transportation becomes available, for example, by obtaining billing information from bus or railway companies at the time of using transportation system IC cards such as senior citizens pass, and accumulating such information as administrative data. The data on the certification for need of support or nursing care is information indicating whether each of a plurality of persons is in need of nursing care or support, and is assumed to include diagnostic data, on the basis of which the need for nursing care or support was certified. The diagnostic data may be not only medical certificates, but also a comprehensive score on basic checklist including the Japanese version of Cardiovascular Health Study (CHS) criteria, a frailty checklist, a locomotive syndrome checklist, etc.

From the standpoint of privacy, such administrative data cannot be obtained and used as it is, and thus it suffices if anonymized data, in which the portions in the administrative data from which individuals can be identified are anonymized, is obtained as administrative data. With this, it is possible to utilize, as the population data, data on the respective age groups in a large population (diagnostic data, behavior information, etc.) included in the administrative data. This can allow for the construction of prediction model 13a that can be used to more accurately predict the future risk of becoming in need of nursing care or support.

### [2.4 Provider 14]

Provider 14 is, for example, a communication interface, and is capable of communicating with mobile terminal 21 of each the plurality of users 20. Provider 14 outputs, to mobile terminal 21 of one user 20, provision information indicating health management support for such one user 20 generated on the basis of the risk predicted by prediction model unit 13. Here, provider 14 may generate the provision information indicating health management support for the one user 20 on the basis of the risk of such one user 20 and the gait information and the terminal usage information on the one user 20, and output the generated provision information to the mobile terminal of such one user 20.

Note that as the provision information indicating health management support, provider 14 may output, to the mobile terminal of the one user 20, the provision information including medical and statistical information that includes a short-term and long-term change amount and the speed of change about the one user 20 obtained by prediction model unit 13.

### [2.5 Updater 15]

Updater 15 includes, for example, a communication interface, a memory, and a processor (microprocessor). Updater 15 can realize the function of updating prediction model 13a by means of the processor executing a predetermined program stored in the memory.

For example, the case can occur where diagnostic data is newly available for the population data that includes the gait information and the terminal usage information on each of the plurality of users 20 stored in storage device 12, after prediction model unit 13 starts the use of the constructed prediction model 13a. In this case, updater 15 is simply required to update prediction model 13a, using the population data that includes the gait information and the terminal usage information on each of a predetermined number of users 20 accumulated in storage device 12 for a new predetermined time period and the diagnostic data, on the basis of which the predetermined number of users 20 were certified as in need of nursing care or support. With this, it is possible for updater 15 to update prediction model 13a to a model that can be used for more accurate prediction of the future risk of becoming in need of nursing care or support.

The case can also occur where the administrative data managed by the administrative body becomes available, after prediction model unit 13 starts the use of constructed prediction model 13a. In this case, it suffices if updater 15 obtains the administrative data managed by the administrative body, which includes the behavior information on a plurality of persons including the usage status of public transportation, and nursing care status information indicating whether a plurality of persons are in need of nursing care or support. Then, it suffices if updater 15 updates prediction model 13a, using the obtained administrative data. This can enable prediction model unit 13 to more accurately predict the future risk of one user 20 becoming in need of nursing care or support, using the updated prediction model 13a.

Furthermore, companies or other entities have medical examination data on each person, and have a grasp of medical expenses, etc. from their health insurance associations. For this reason, the case can occur where, the medical examination data held by the companies or other entities and the medical expenses, etc. from the health insurance associations become available, after prediction model unit 13 starts the use of the constructed prediction model 13a. In this case, it suffices if updater 15 obtains the medical examination data held by the companies or other entities and the medical expenses, etc. from the health insurance associations. Then, it suffices if updater 15 updates prediction model 13a, using the obtained medical examination data held by the company or other entities and the medical expenses, etc. from the health insurance associations. This can enable prediction model unit 13 to more accurately predict the future risk of one user 20 becoming in need of nursing care or support, using the updated prediction model 13a.

Note that updater 15 may obtain prediction model 13a that has been updated as in the foregoing manner, and update prediction model 13a in prediction model unit 13.

### [3 Operation of Health Management Support System 100]

The following describes an example of the operation performed by health management support system 100 having the configuration described above.

FIG. 3 is a flowchart showing an example of the processing of the health management support method performed by health management support system 100 according to Embodiment 1.

First, health management support system 100 obtains, from each mobile terminal 21 of the plurality of users 20, the personal data including the gait information and the terminal usage information on each user 20, at predetermined time intervals (S1). A non-limiting example of the predetermined time intervals is every day, but may be every other day or every few days.

Next, health management support system 100 accumulates, in storage device 12, the population data that includes the personal data on each of the plurality of users 20 obtained in step S1 (S2). A time period during which the population data is accumulated may be, but is not limited to, a certain length of period or longer, such as one year or longer. The time period during which the population data is accumulated is preferably a time period in which it is possible to examine temporal changes in the personal data that includes the gait information and the terminal usage information.

Next, using prediction model 13a, health management support system 100 predicts the future risk of one user 20 becoming in need of nursing care or support from the time-series personal data on such one user 20 accumulated in storage device 12 for the predetermined time period (S3). Prediction model 13a is a model that enables the prediction of such risk of an individual, by applying the population data to the personal data that includes temporal changes.

Next, health management support system 100 outputs, to mobile terminal 21 of the one user 20, the provision information indicating health management support for the one user 20 generated on the basis of the risk predicted in step S3 (S4).

### [4 Effects, etc.]

As described above, according to Embodiment 1, it is possible to predict the future risk of one user 20 becoming in need of nursing care or support, from the gait information and the terminal usage information obtained from mobile terminal 21 of each of the plurality of users 20 and accumulated. It is then possible to provide, to the one user 20, the provision information indicating health management support for such one user 20 generated on the basis of the predicted risk.

With this, it is possible for user 20 to obtain the provision information, such as health management support, simply through a daily use of mobile terminal 21, without having to install any special software in mobile terminal 21 of such user 20. Consequently, user 20 becomes aware of the need for changing his/her behavior to avoid the risk and can change his/her behavior in response to the reception of the provision information. It is thus possible for user 20 to avoid becoming in need of support or nursing care in the future.

In addition, by operating health management support system 100 according to the present embodiment, data on changes in the condition of each of the plurality of users 20 who have changed their behaviors in response to the reception of the provision information will be further accumulated. It is thus estimated that the accuracy of risks to be predicted will further increase.

### (Working Example)

With the implementation of the Basic Act on the Advancement of Public and Private Sector Data Utilization, it is becoming possible for many municipalities to use administrative data. At present, however, it cannot be said that such administrative data is fully available or utilized.

In the present working example, a health management support method is described for the case where the administrative data becomes available or utilized in the future.

FIG. 4 is a diagram conceptually showing the health management support method according to the working example of Embodiment 1.

FIG. 4 conceptually illustrates an example case where, for example, prediction model 13a is updated using the administrative data, after prediction model 13a is constructed using the gait information and the terminal usage information on each of a predetermined number of users 20 (population data) accumulated in storage device 12 (DB 12a) for a predetermined time period in the past. In this case, as shown in FIG. 4, prediction model 13a is constructed and updated in a predetermined organization called a secretariat.

First, a management secretariat (person) in the secretariat analyzes and examines the population data that includes the gait information and the terminal usage information of the predetermined time period in the past to extract the physical information, the social information, and the mental information on a predetermined number of users. Subsequently, the management secretariat can construct prediction model 13a using the items of information extracted from the population data, and the diagnostic data, on the basis of which the predetermined number of users were certified as in the need of nursing care or support. The management secretariat is simply required to transmit (provide) the constructed prediction model 13a to cloud 10.

The management secretariat is not allowed to obtain and use the administrative data as it is from the standpoint of privacy. For this reason, the management secretariat is simply required to obtain, as the administrative data, anonymized data in which the portions in the administrative data from which the individuals can be identified (name, date of birth, etc.) are anonymized. Next, the management secretariat analyzes and examines the population data such as the behavior information included in the administrative data to extract the physical information, the social information, and the mental information on the users included in the administrative data. The management secretariat is simply required to update prediction model 13a and transmit (provide) the updated prediction model 13a to cloud 10, by further using the diagnostic data, included in the information indicating the need for support/nursing care, on the basis of which the need for nursing care or support was certified.

In the future, it can be considered possible to utilize the following example items of administrative data to construct and update prediction model 13a: medical fee receipt data; nursing care fee receipt data, nursing care certification survey sheets; medical examination data; public assistance recipient survey sheets (personal data); vaccination status; emergency service dispatch information and injury/disease information; and lists of dates of moving in, moving out, death, etc. It is considered further possible to utilize the foregoing medical examination data held by companies or other entities to construct and update prediction model 13a.

### [Embodiment 2]

The following describes a health management support system according to Embodiment 2 with reference to the drawings.

### [5 Configuration of Health Management Support System]

FIG. 5 is a block diagram showing an example of the configuration of health management support system 101 according to Embodiment 2. Health management support system 101 shown in FIG. 5 includes generative artificial intelligence (AI) 14a in provider 14, in addition to the elements of health management support system 100 according to Embodiment 1. The following describes health management support system 101 according to Embodiment 2, focusing on the point added from health management support system 100 according to Embodiment 1 (stated differently, generative AI 14a), and the same points will not be described. Note that health management support system 101 shown in FIG. 5 realizes, for example, cloud 10 shown in FIG. 1, using the elements thereof in part or in whole.

### [5.1 Generative AI 14a]

Generative AI 14a is, for example, a generative AI engine, such as ChatGPT^{®} 4.0 developed by OpenAI, LLaMA developed by Mata, Bard developed by Google, etc. Generative AI 14a receives a question from one user 20 about the health condition of such one user 20 and generates a response to the question, using prediction model 13a, to output the response to mobile terminal 21. Note that the response outputted by generative AI 14a is a response in a conversational form. More specific examples of the response are described later.

Using prediction model 13a, generative AI 14 provides a response that includes organizing the personal data on the one user 20 and generating a prediction result indicating the one user 20's future condition of the development into at least one of frailty, locomotive syndrome, or dementia. Furthermore, using prediction model 13a, generative AI 14a provides a response recommending to take measures to inhibit the development of the one user 20 into at least one of frailty, locomotive syndrome, or dementia.

Organizing the personal data is, for example, generating a graph, etc. showing the physical information or the mental information on the one user 20 accumulated by generative AI 14a on a daily basis. Further, in organizing the personal data, generative AI 14a may add, to the graph, etc., the future physical information, mental information, etc. of the one user 20 that are predicted using prediction model 13a.

### [6 Operation of Health Management Support System 101]

The following describes an example of the operation performed by health management support system 101 having the foregoing configuration.

FIG. 6 is a flowchart showing an example of the processing of the health management support method performed by health management support system 101 according to Embodiment 2. Note that the processes of step S1 to step S3 performed by health management support system 101 shown in FIG. 6 are the same as the processes of step S1 to step S3 performed by health management support system 100 shown in FIG. 3, and thus will not be described.

Subsequent to step S3, one user 20 enters, to generative AI 14a, a question about the health condition of such one user 20 (S5). The question entered by the one user 20 may be an arrangement of a plurality of words or a question formed of a long sentence.

Next, on the basis of the question from the one user 20, generative AI 14a organizes the personal data on the one user 20, generates a prediction result of the future health condition of the one user 20 or measures to be taken against the prediction result, using prediction model 13a, and transmits the resultant to mobile terminal 21 of the one user 20 (S6).

### [7 Example Display on Mobile Terminal 21]

FIG. 7 is a diagram for describing input/output regions on mobile terminal 21. Here, the input/output regions are regions on the display included in mobile terminal 21, and includes not only the display region, but also an input region in the form of a touch panel on the display.

As shown in FIG. 7, mobile terminal 21 includes, as the input/output regions, input region 31, graph display region 32, response display region 33, and measures display region 34.

Input region 31 is a region to which one user 20 enters a question about the health condition of such one user 20. A method of entering the question may be a manual input method in which the one user 20 enters the question by operating, for example, a keyboard or a touch panel, or may be a voice input method in which the one user 20 speaks a question loud, and mobile terminal 21 recognizes such voice of the one user 20 and enters the question.

Graph display region 32 is a region for displaying a graph, etc. generated by generative AI 14a by organizing the personal data on the one user 20, using prediction model 13a, in response to the reception of the question from the one user 20 about the health condition of such one user 20. The graph, etc. to be displayed shows data accumulated in storage device 12, which is, for example, the physical information such as a daily walking time, a daily number of steps, or a walking speed of one user 20, the frequency with which the one user 20 exchanges emails using mobile terminal 21, the mental information such as sleep information, etc. The graph, etc. displayed in graph display region 32 may include not only the data accumulated in storage device 12, but also the future physical information, mental information, etc. of the one user 20 predicted, using prediction model 13a.

Response display region 33 is a region for displaying a response to the question from the one user 20 generated by generative AI 14a, using prediction model 13a, in response to the reception of the question about the health condition of the one user 20. The contents of the response may be, for example, a detailed explanation for changes in the physical information, the mental information, etc. of the one user 20 accumulated in storage device 12, or may be a detailed explanation for the future health condition of the one user 20 predicted from the changes in the physical information, the mental information, etc. of the one user 20 accumulated in storage device 12. The contents of the response may also be a detailed explanation for the graph displayed in graph display region 32.

Measures display region 34 is a region for displaying the measures to be taken against the future health condition of the one user 20 predicted by generative AI 14a, using prediction model 13a, in response to the reception of the question about the health condition of the one user 20. The measures are, for example, a response recommending actions that should be taken by the one user 20 to inhibit the development into at least one of frailty, locomotive syndrome, or dementia.

### [8 Specific Example of Display on Mobile Terminal 21]

FIG. 8A is a diagram for describing the input/output regions on mobile terminal 21 when one user 20 enters a question to mobile terminal 21.

As shown in FIG. 8A, when the one user 20 enters, to input region 31, "What is my future health condition and the measures for it?", generative AI 14a receives "What is my future health condition and the measures for it?".

FIG. 8B is a diagram for describing the input/output regions on mobile terminal 21 when generative AI 14a responds to the question from the one user 20.

As shown in FIG. 8B, when receiving "What is my future health condition and the measures for it?", generative AI 14a generates a response to the question, using prediction model 13a, and displays the response in graph display region 32, response display region 33, and measures display region 34. For example, in graph display region 32, graphs are displayed that represent, in the solid lines, the average number of daily steps and the average number of daily emails of the one user 20 accumulated in storage device 12. The graphs further show, in the dotted lines, the future average number of daily steps and the future average number of daily emails of the one user 20 predicted using prediction model 13a. Also, response display region 33 shows a response to the question from the one user 20, such as "From 2020 to 2022, the average number of daily steps has been on a downward trend. If this trend continues, it may become difficult for you to walk alone in five years". Also, measures display region 34 shows the measures responsive to the question from the one user 20, such as "The following measures are suggested: (1) take a walk for 10 minutes a day; and (2) do stretching and radio exercises".

### [9 Effects, etc.]

As described above, according to Embodiment 2, it is possible for health management support system 101 to receive a question from one user 20 about his/her personal health condition, and provide advice to the one user 20. This enables the one user 20 to carry out the advice from health management support system 101, as a result of which such one user 20 is able to avoid becoming in need of support or nursing care in the future.

### (Added Notes)

With the foregoing embodiments and others, the technologies described below are disclosed.

(Technology 1) A health management support system including: an obtainer that obtains personal data from each terminal device of a plurality of users at predetermined time intervals, the personal data including gait information on the user and terminal usage information indicating a usage status of the terminal device of the user; a storage device that accumulates population data including the personal data on each of the plurality of users obtained by the obtainer; a prediction model unit that predicts a future risk of one user becoming in need of nursing care or support, using a prediction model, from time-series personal data that is the personal data on the one user accumulated in the storage device for a predetermined time period, the one user being included in the plurality of users, the prediction model being a model for predicting the future risk by applying the population data to the personal data that includes temporal changes; and a provider that outputs, to the terminal device of the one user, provision information that is generated based on the future risk predicted and indicates health management support for the one user.

As in the foregoing manner, it is possible to predict the future risk of one user 20 becoming in need of nursing care or support from the gait information and the terminal usage information (i.e., personal data) obtained from mobile terminal 21 of each of users 20 and accumulated. This enables each of the plurality of users 20 to avoid becoming in need of support or nursing care in the future.

To be more specific, it is possible to predict the future risk of the one user 20 becoming in need of nursing care or support, using the prediction model, from the personal data, which is obtained from mobile terminal 21 of each user 20 and which can be said to be time-series daily lifestyle data on the individual accumulated. It is then possible to provide, to the one user 20, the provision information indicating health management support for such one user 20 generated on the basis of the predicted risk.

Meanwhile, all what user 20 needs to do is to provide, to the health management support system, the gait information and the terminal usage information that are obtained through a daily use of mobile terminal 21, without having to install any special software in mobile terminal 21 of such user 20. With this, it is possible for user 20 to receive the provision information indicating health management support that is generated on the basis of the future risk of becoming in need of nursing care or support. User 20 becomes aware of the need for changing his/her behavior to avoid the future risk of becoming in need of nursing care or support and can change his/her behavior in response to the reception of the provision information. It is a reality that user 20 cannot have a good grasp of him/herself. In particular, user 20 cannot be aware of gradually developing vitality decline, daily activities decline, mental and psychological vitality decline, social activity decline, etc. However, user 20 becomes aware of these in response to the reception of the provision information and the need for changing his/her behavior.

Consequently, it is possible for user 20 to avoid becoming in need of support or nursing care in the future. Stated differently, the health management support system according to one aspect of the present disclosure enables each of users 20 to avoid becoming in need of support or nursing care in the future. In addition, it is possible to prompt the plurality of users 20 to change their behaviors as described above, thus contributing to inhibiting the number of elderly persons who will become in need of support or nursing care in the future.

(Technology 2) The health management support system according to technology 1, wherein the prediction model is constructed from the population data and diagnostic data, the population data including the gait information and the terminal usage information on each of a predetermined number of users accumulated in the storage device for a predetermined time period in past, the diagnostic data being data based on which each of the predetermined number of users was certified as in need of nursing care or support.

As in the foregoing manner, it is possible to obtain the population data from the gait information and the terminal usage information accumulated for each of the predetermined number of users 20. It is then possible to construct the prediction model from the obtained population data and the diagnostic data. With this, it is possible to obtain the prediction model for predicting the future risk of an individual becoming in need of nursing care or support by applying the population data to the personal data that includes temporal changes.

(Technology 3) The health management support system according to technology 2, wherein the prediction model is constructed from physical information indicating a physical condition of each of the predetermined number of users, social information indicating a social contact condition of the user, and mental information indicating a mental condition of the user, and the diagnostic data on the user, the physical information, the social information, and the mental information having been extracted from the population data.

As described above, it is possible to construct the prediction model, using the physical information, the social information, and the mental information extracted from the population data.

(Technology 4) The health management support system according to technology 3, wherein the physical information is the gait information on each of the predetermined number of users, and the gait information indicates a relation of at least two of a daily walking time, a daily number of steps, a walking speed, a stride length, a total number of stairs climbed, or a walking distance.

(Technology 5) The health management support system according to technology 3 or 4, wherein the social information is information on an interaction with another person performed by each of the predetermined number of users using the terminal device, and the information on the interaction includes at least one of a frequency with which the user exchanges an email using the terminal device, a length of a sentence included in the email, a total number of persons with whom the user exchanges the email, or a usage rate of various software and applications in the terminal device.

(Technology 6) The health management support system according to any one of technologies 3 to 5, wherein the mental information includes at least one of sleep information or mindfulness information on each of the predetermined number of users.

(Technology 7) The health management support system according to any one of technologies 2 to 6, further includes: an updater that obtains administrative data managed by an administrative body, and updates the prediction model, using the administrative data obtained, the administrative data including behavior information on each of a plurality of persons including a usage status of public transportation and nursing care status information indicating whether each of the plurality of persons is in need of nursing care or support.

As described above, the administrative data is obtained to update the prediction model. With this, it is possible to update the prediction model to a model that can be used for more accurate prediction of the future risk of becoming in need of nursing care or support.

(Technology 8) The health management support system according to any one of technologies 1 to 7, wherein the provider generates the provision information indicating the health management support for the one user, based on the future risk of the one user, and the gait information and the terminal usage information on the one user, and outputs the provision information to the terminal device of the one user.

With this, it is possible to cause user 20 who has received the provision information to be aware of the need for changing his/her behavior to avoid the future risk of becoming in need of nursing care or support. This enables user 20 to change his/her behavior, as a result of which user 20 is able to avoid becoming in need of support or nursing care in the future.

(Technology 9) The health management support system according to any one of technologies 1 to 8, wherein the health management support is a measure to inhibit development into at least one of frailty, locomotive syndrome, or dementia, and the measure includes recommending to treat or manage an injury or a disease that causes the development into at least one of frailty, locomotive syndrome, or dementia, or to do exercise to inhibit the development into at least one of frailty, locomotive syndrome, or dementia.

With this, it is possible to cause user 20 who has received the provision information to be aware of the need for getting treatment and doing management and exercise for inhibiting the development into frailty, locomotive syndrome, and dementia. This causes user 20 to get treatment and do management and exercise, thereby enabling user 20 to avoid becoming in need of support or nursing care in the future.

(Technology 10) The health management support system according to any one of technologies 1 to 9, wherein the provider includes a generative artificial intelligence (AI), and the generative AI receives, from the one user, a question about a health condition of the one user to generate a response to the question, using the prediction model, and outputs the response.

With this, it is possible for health management support system 101 to receive a question about a personal health condition from one user 20 and provide advice to such one user 20. This enables the one user 20 to carry out the advice from health management support system 101, as a result of which such one user 20 is able to avoid becoming in need of support or nursing care in the future.

(Technology 11) The health management support system according to technology 10, wherein the health management support is a measure to inhibit development into at least one of frailty, locomotive syndrome, or dementia, and the response includes: organizing the personal data on the one user; generating a prediction result indicating a future condition of the development of the one user into at least one of frailty, locomotive syndrome, or dementia; or recommending the measure to inhibit the development into at least one of frailty, locomotive syndrome, or dementia.

With this, it is possible for health management support system 101 to receive a question about a personal health condition from one user 20 and provide, to such one user 20, advice in a more comprehensible, explanatory form or a in conversational form. This enables the one user 20 to carry out the advice from health management support system 101, as a result of which such one user 20 is able to avoid becoming in need of support or nursing care in the future.

(Technology 12) A health management support method including: obtaining personal data from each terminal device of a plurality of users at predetermined time intervals, the personal data including gait information on the user and terminal usage information indicating a usage status of the terminal device of the user; accumulating, in a storage device, population data including the personal data on each of the plurality of users obtained in the obtaining; predicting a future risk of one user becoming in need of nursing care or support, using a prediction model, from time-series personal data that is the terminal usage information on the one user and accumulated in the storage device for a predetermined time period, the one user being included in the plurality of users, the prediction model being a model for predicting the future risk by applying the population data to the personal data that includes temporal changes; and outputting, to the terminal device of the one user, provision information that is generated based on the future risk predicted and indicates health management support for the one user.

### (Other Possible Embodiments)

The health management support system and the health management support method of the present disclosure have been described in the foregoing embodiments and working example, but the processes are not limited to being performed by specific entities and devices. The processes may be performed by, for example, a processor embedded in a specific device that is locally located. The processes may also be performed by a cloud (cloud server) disposed in a location different from the location of the local device.

Note that the present disclosure is not limited to the foregoing embodiments and so forth. For example, another embodiment realized by freely combing elements described in this description or by removing some of the elements may serve as an embodiment of the present disclosure. Also, the present disclosure also includes a variation obtained by making various modifications to the foregoing embodiments that can be conceived by those skilled in the art without departing from the essence of the present disclosure, that is, the meaning indicated by the language recited in Claims.

Also, the present disclosure further includes the cases described below.

(1) Each of the foregoing devices is, more specifically, a computer system that includes a microprocessor, a ROM, a RAM, a hard disk unit, a display unit, a keyboard, a mouse, etc. The RAM or the hard disk unit stores a computer program. The microprocessor's operating in accordance with the computer program enables each of the devices to achieve its function. Here, the computer program is configured, using a combination of a plurality of command codes representing instructions given to the computer to achieve a predetermined function.
(2) One or more, or all of the elements included in the foregoing devices may be configured in the form of a single system large scale integration (LSI). The system LSI is a super-multifunctional LSI that is manufactured by integrating a plurality of elements onto a single chip. The system LSI is, more specifically, a computer system that is configured using a microprocessor, a ROM, a RAM, etc. The RAM stores a computer program. The microprocessor's operating in accordance with the computer program enables the system LSI to achieve its function.
(3) One or more, or all of the elements included in at least one of the foregoing devices may be implemented in the form of an IC card removable from the device or a single module. The IC card or the module is a computer system that includes a microprocessor, a ROM, a RAM, etc. The IC card or the module may include the foregoing super-multifunctional LSI. The microprocessor's operating in accordance with the computer program enables the IC card or the module to achieve its function. Such IC card or the module may be tamper resistant.
(4) The present disclosure may be the method described above. The present disclosure may also be a computer program that enables such method to be implemented by means of a computer, or digital signals that form the computer program.
(5) The present disclosure may be configured by means of recording the computer program or the digital signals on a computer-readable recording medium such as a flexible disk, a hard disk, a compact (CD)-ROM, a DVD, a DVD-ROM, a DVD-RAM, a Blu-ray^{®} disc (BD), and a semiconductor memory. The present disclosure may also be configured in the form of the digital signals recorded in such recording medium.

The present disclosure may be configured by means of transmitting the computer program or the digital signals via, for example, a telecommunication line, a wireless or wired communication line, a network represented by the Internet, and data broadcasting.

The present disclosure may also be a computer system including a microprocessor and a memory, where the memory stores the computer program and the microprocessor operates in accordance with the computer program.

The present disclosure may be implemented by means of transmitting the program or the digital signals recorded on the recording medium or transmitting the program or the digital signals via, for example, the network, thereby enabling another independent computer system to carry out the present disclosure.

### [Industrial Applicability]

The present disclosure is applicable for use as a health management support system, a health management support method, and a program for supporting the health management of each user, using data obtained from a mobile terminal, such as a smartphone, used by each user.

### [Reference Signs List]

- 10: cloud
- 11: obtainer
- 12: storage device
- 12a, 12b: DB
- 13: prediction model unit
- 13a: prediction model
- 14: provider
- 14a: generative AI
- 15: updater
- 20: user
- 21: mobile terminal
- 31: input region
- 32: graph display region
- 33: response display region
- 34: measures display region
- 100, 101: health management support system

## Claims

1. A health management support system comprising:
an obtainer that obtains personal data from each terminal device of a plurality of users at predetermined time intervals, the personal data including gait information on the user and terminal usage information indicating a usage status of the terminal device of the user;
a storage device that accumulates population data including the personal data on each of the plurality of users obtained by the obtainer;
a prediction model unit that predicts a future risk of one user becoming in need of nursing care or support, using a prediction model, from time-series personal data that is the personal data on the one user accumulated in the storage device for a predetermined time period, the one user being included in the plurality of users, the prediction model being a model for predicting the future risk by applying the population data to the personal data that includes temporal changes; and
a provider that outputs, to the terminal device of the one user, provision information that is generated based on the future risk predicted and indicates health management support for the one user.

2. The health management support system according to claim 1,
wherein the prediction model is constructed from the population data and diagnostic data, the population data including the gait information and the terminal usage information on each of a predetermined number of users accumulated in the storage device for a predetermined time period in past, the diagnostic data being data based on which each of the predetermined number of users was certified as in need of nursing care or support.

3. The health management support system according to claim 2,
wherein the prediction model is constructed from physical information indicating a physical condition of each of the predetermined number of users, social information indicating a social contact condition of the user, and mental information indicating a mental condition of the user, and the diagnostic data on the user, the physical information, the social information, and the mental information having been extracted from the population data.

4. The health management support system according to claim 3,
wherein the physical information is the gait information on each of the predetermined number of users, and
the gait information indicates a relation of at least two of a daily walking time, a daily number of steps, a walking speed, a stride length, a total number of stairs climbed, or a walking distance.

5. The health management support system according to claim 3,
wherein the social information is information on an interaction with another person performed by each of the predetermined number of users using the terminal device, and
the information on the interaction includes at least one of a frequency with which the user exchanges an email using the terminal device, a length of a sentence included in the email, a total number of persons with whom the user exchanges the email, or a usage rate of various software and applications in the terminal device.

6. The health management support system according to claim 3,
wherein the mental information includes at least one of sleep information or mindfulness information on each of the predetermined number of users.

7. The health management support system according to any one of claims 2 to 5, further comprising:
an updater that obtains administrative data managed by an administrative body, and updates the prediction model, using the administrative data obtained, the administrative data including behavior information on each of a plurality of persons including a usage status of public transportation and nursing care status information indicating whether each of the plurality of persons is in need of nursing care or support.

8. The health management support system according to any one of claims 1 to 5,
wherein the provider generates the provision information indicating the health management support for the one user, based on the future risk of the one user, and the gait information and the terminal usage information on the one user, and outputs the provision information to the terminal device of the one user.

9. The health management support system according to any one of claims 1 to 5,
wherein the health management support is a measure to inhibit development into at least one of frailty, locomotive syndrome, or dementia, and
the measure includes recommending to treat or manage an injury or a disease that causes the development into at least one of frailty, locomotive syndrome, or dementia, or to do exercise to inhibit the development into at least one of frailty, locomotive syndrome, or dementia.

10. The health management support system according to any one of claims 1 to 5,
wherein the provider includes a generative artificial intelligence (AI), and
the generative AI receives, from the one user, a question about a health condition of the one user to generate a response to the question, using the prediction model, and outputs the response.

11. The health management support system according to claim 10,
wherein the health management support is a measure to inhibit development into at least one of frailty, locomotive syndrome, or dementia, and
the response includes: organizing the personal data on the one user; generating a prediction result indicating a future condition of the development of the one user into at least one of frailty, locomotive syndrome, or dementia; or recommending the measure to inhibit the development into at least one of frailty, locomotive syndrome, or dementia.

12. A health management support method comprising:
obtaining personal data from each terminal device of a plurality of users at predetermined time intervals, the personal data including gait information on the user and terminal usage information indicating a usage status of the terminal device of the user;
accumulating, in a storage device, population data including the personal data on each of the plurality of users obtained in the obtaining;
predicting a future risk of one user becoming in need of nursing care or support, using a prediction model, from time-series personal data that is the terminal usage information on the one user and accumulated in the storage device for a predetermined time period, the one user being included in the plurality of users, the prediction model being a model for predicting the future risk by applying the population data to the personal data that includes temporal changes; and
outputting, to the terminal device of the one user, provision information that is generated based on the future risk predicted and indicates health management support for the one user.

13. A program for causing a computer to execute:
obtaining personal data from each terminal device of a plurality of users at predetermined time intervals, the personal data including gait information on the user and terminal usage information indicating a usage status of the terminal device of the user;
accumulating, in a storage device, population data including the personal data on each of the plurality of users obtained in the obtaining;
predicting a future risk of one user becoming in need of nursing care or support, using a prediction model, from time-series personal data that is the gait information and the terminal usage information on the one user and accumulated in the storage device for a predetermined time period, the one user being included in the plurality of users, the prediction model being a model for predicting the future risk by applying the population data to the personal data that includes temporal changes; and
outputting, to the terminal device of the one user, provision information that is generated based on the future risk predicted and indicates health management support for the one user.
